# EUROPEAN PATENT SPECIFICATION

(11) **EP 2 091 520 B1**
(45) Date of publication and mention of the grant of the patent: **22.02.2012**
(21) Application number: 07822468.0
(22) Date of filing: 09.11.2007
(51) Int. Cl.: A61K 9/16, A61K 9/48, A61K 31/00, A61K 9/28, A61K 9/20, A61K 31/095, A61K 31/66

(54) **ORAL DOSAGE FORM COMPRISING TRI-SUBSTITUTED GLYCEROL COMPOUNDS**
ORALE DOSIERFORM MIT TRISUBSTITUIERTEN GLYCERIN-VERBINDUNGEN
FORME GALÉNIQUE DESTINÉE À LA VOIE ORALE COMPRENANT DES COMPOSÉS DE GLYCÉROL TRISUBSTITUÉS

(30) Priority: 10.11.2006 US 858157 P
(43) Date of publication of application: 26.08.2009
(73) Proprietor: Alphaptose Gmbh, 20149 Hamburg (DE)
(72) Inventor: RICHTER, Wolfgang, 81243 München (DE); WEBER, Lutz, 82110 Germering (DE)
(74) Representative: Danner, Stefan
(86) International application number: PCT/EP2007/062180
(87) International publication number: WO 2008/055996

(56) References cited:
- WO-A-90/14829
- WO-A-99/30690
- DE-A1- 19 822 509
- US-A1- 2004 138 263
- PINCHUK A N ET AL: "SYNTHESIS OF ALKYL GLYCEROPHOSPHOLIPIDS THROUGH 1-0-BENZYL-2-0-METHYL-rac-GLYCEROL" PHARMACEUTICAL CHEMISTRY JOURNAL, CONSULTANTS BUREAU, NAW YORK, NY, US, vol. 26, 1992, pages 174-176, XP008087544 ISSN: 0091-150X

## Description

The present invention relates to pharmaceutical solid dosage forms for oral administration comprising a tri-substituted glycerol compound or a pharmaceutically acceptable salt thereof wherein the dosage form is selected from the group consisting of tablets, pills, capsules granulates, pellets, powders and dragees. . The invention also relates to a corresponding method for preparing such dosage forms as well as to their use as medicaments for the treatment of cancer and immune diseases.

The tri-substituted glycerol compounds used in the present invention belong to the class of synthetic ether-linked alkyl-lysophospholipids. Since these lipids are known to have an anti-cancerogenic activity, they are also collectively named "anti-tumor ether lipids" (reviewed, e.g., by Arthur, G., and Bittman, R. (1998) Biochim. Biophys. Acta 1390, 85-102; Jendrossek, V., and Handrick, R. (2003) Curr. Med. Chem. Anti-Canc. Agents 3, 343-353; Mollinedo, F. et al. (2004) Curr. Med. Chem. 11, 3163-3184).

Aside from their anti-tumor activity, these ether lipids are believed to be involved in a variety of other physiological processes such as inflammation, the immune response or allergic reactions. It is established in the art that these ether lipids can be used as medicaments for the treatment of various immune diseases (cf., for example, the International Patent Applications WO 87/01257 and WO 90/14829, respectively).

1-*O*-octadecyl-2-*O*-methyl-glycero-3-phosphocholine (also referred to as ET-18-OCH3, AP-121 or edelfosine) is considered to be the prototype of the anti-tumor ether lipids. 1-*O*-octadecyl-2-*O*-methyl-glycero-3-phosphocholine represents a synthetic analogue of the platelet activating factor (PAF; 1-O-alkyl-2-acetyl-sn-glycero-3-phosphocholine), a potent phospholipid activator and mediator of many leukocyte functions, including platelet aggregation, inflammation, and anaphylaxis. Unlike most conventional chemotherapeutic drugs, the anti-tumor ether lipids do not directly target cellular DNA but rather affect the plasma membrane lipid composition and/or interfere with various signal transduction pathways. Two major cellular targets of 1-*O*-octadecyl-2-*O*-methyl-glycero-3-phosphocholine have been identified so far, namely the CTP: phosphocholine cytidylyl-transferase (CCT; EC 2.7.7.15) and the death receptor Fas (also known as APO-1 or CD95). In a recent study, 1-*O-*octadecyl-2-*O*-methyl-glycero-3-phosphocholine has been further demonstrated to target two different sub-cellular structures in a cell type-dependent manner, namely cell surface lipid rafts in leukemic cells and the endoplasmic reticulum in solid tumor cells, and to affect processes taking place in both structures that eventually induce lipid raft- and endoplasmic reticulum-mediated cell death, respectively (Nieto-Miguel, T. et al. (2006) J. Biol. Chem. 281, 14833-14840).

Cancer chemotherapy generally aims to slow the growth of, or destroy, cancer cells while avoiding collateral damage to surrounding cells and tissues. Consequently, the most effective anticancer agents are those that are able to selectively target cancer cells while leaving normal cells relatively unaffected. Synthetic ether-lipids have been shown to be effective as tumor agents, for example, in order to decrease or to stop tumor progression, i.e. to stabilize the "status quo" of the condition, or even to reduce the size of tumors in mammals. It has been found that 1-*O*-octadecyl-2-*O-*methyl-glycero-3-phosphocholine is particularly suitable for the treatment of different types of tumors such as brain tumors or mamma carcinomas (cf., for example, the German Patent DE 2619686 as well as the International Patent Applications WO 99/59599 and WO 00/01392, respectively).

Several mechanisms of action have been proposed for the toxicity of ether-lipids towards cancer cells, including the cells' lack of alkyl cleavage enzymes. The resultant inability to hydrolyze the ether-lipids leads to their intracellular accumulation and the consequent damage of cell membrane lipid organization. Other potential mechanisms of ether-lipid action include effects on levels of intracellular protein phosphorylation, and disruption of cellular lipid metabolism. Normal cells typically possess the means to avoid or overcome the potentially toxic effects of ether-lipids, while cancer cells do not.

The anti-tumor activity of these synthetic ether lipids has been experimentally proven in several animal tumor models. However, their clinical use is often hampered by systemic cytotoxic effects including hemolysis (particularly observed in the gastrointestinal tract but also *inter alia* in lung, liver or kidney).

1-*O*-octadecyl-2-*O*-methyl-glycero-3-phosphocholine and other synthetic ether-lipids can be administered to patients by using the intravenous route. In this context, it was found that the intravenous administration of a liposomal formulation is advantageous in order to improve therapeutic efficacy while markedly reducing nonspecific toxicity *in vivo* (see, for example, Ahmad, I. et al. (1997) Cancer Res. 57, 1915-1921).

The International Patent Application WO 91/09590 describes a pharmaceutical preparation of 1-*O*-octadecyl-2-*O*-methyl-glycero-3-phosphocholine for intravenous administration that contains a lipophilic oil-in-water emulsion that can be used to administer high doses of the compound without adverse side effects.

The German patent application DE19822509 discloses liquid compositions
comprising octadecyl-2-methyl-sn-glycero-3-phosphocholin (edelfosin, L-form, D-form or razemat) for the treatment of brain cancer.

The international patent application WO9930690 relates to solid flakes which are orally administered for the treatment of cancer comprising a pharmaceutical active such as edelfosine.

However, it is also known in the art that certain ether phospholipid and carbamoyl salts while exhibiting benefits to a patient as competitive inhibitors of PAF or tumor growth with single or repeated injections, cause detrimental effects in the area of the injection. These detrimental effects are evident as lysis of red blood cells, severe edema, inflammation, and injection site-necrosis. These adverse effects are also called "detergent" effects. Where repeated injections are required, these detrimental effects are particularly disadvantageous as they render the sites of administration unsuitable and require fresh sites. Since the number of suitable sites on a patient is limited, it would be highly desirable to avoid said detrimental effects associated with intravenous administration of 1-*O*-octadecyl-2-*O*-methyl-glycero-3-phosphocholine.

More recently, it has been shown that it is also possible to administer 1-*O*-octadecyl-2-*O*-methyl-glycero-3-phosphocholine orally together with a liquid drinkable vehicle. In the International Patent Application WO 99/59599, it is described that 1-*O*-octadecyl-2-*O*-methyl-glycero-3-phosphocholine can be administered together with water-based vehicles containing at least 3% (w/w) fat and/or protein such as soups (especially thickened soups), eggnog and other conventional beverages. Milk-based vehicles are also suitable, such as milk, milk substitute, yogurt, kefir and the like. It is tempting to speculate that an efficient binding of 1-*O*-octadecyl-2-*O-*methyl-glycero-3-phosphocholine to the proteins and/or other lipids "mask" the ether-lipid thus resulting in a reduction of adverse side effects.

Nevertheless, in 10-20% of the patients treated with such water- and/or milk-based vehicles significant gastrointestinal incompatibilities (corresponding to WHO toxicity grades III and IV, respectively) have been observed that are associated with loss of appetite, nausea and/or vomiting, diarrhea, constipation or the like (see, e.g., Drings, P. et al. (1992) Onkologie 15, 375-382).

Furthermore, notwithstanding the additional problem of food allergies (such as lactose incompatibility) it is also not convenient for a patient to take the medicament with considerable amounts of food or drinks several times a day. Furthermore, it is evident that e.g. a milk-based medicament needs to be prepared every time immediately before administration. This is not only time consuming and non-practical, but may also be an element of uncertainty with respect to the dose amount, since the preparation of the medicament requires accurate weighing and intense mixing. Finally, it must also be ensured that the patient takes the medicament completely to ensure uptake of the complete dose amount.

Thus, there still remains a need for an alternative oral dosage form comprising 1-*O-*octadecyl-2-*O*-methyl-glycero-3-phosphocholine or a related tri-substituted glycerol compound that overcomes the above limitations. In particular, there is a need for a dosage form, which is in solid form, allows for an easy and convenient administration and provides the required pharmaceutical efficacy with respect to the treatment of cancer and other diseases. Since the uptake of 1-*O*-octadecyl-2-*O-*methyl-glycero-3-phosphocholine or related tri-substituted glycerol compounds takes place in the colon, it would be most desirable to have a solid enteric pharmaceutical dosage form that passes the stomach without being disintegrated.

Accordingly, it is an object of the present invention to provide such a pharmaceutical solid dosage form for oral administration.

This object is achieved by the pharmaceutical dosage form having the features of independent claim 1. Some of the preferred embodiments of the present invention are defined by the subject matter of the dependent claims.

According to the present invention, it has been found that it is possible to formulate solid oral dosage forms selected from the group consisting of tablets, pills, capsules, granulates, pellets, powders and dragees containing tri-substituted glycerol compounds such as 1-*O-*octadecyl-2-*O*-methyl-glycero-3-phosphocholine which are suitable for treating cancer or immune diseases, and which allow for a precise dosing and a convenient taking of the medicament. The inventive oral dosage form provides the desired efficacy or the required bioavailability of the active agent when administered to patients.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%.

In a first aspect, the present invention relates to pharmaceutical solid dosage forms for oral administration comprising a tri-substituted glycerol compound according to formula (I) 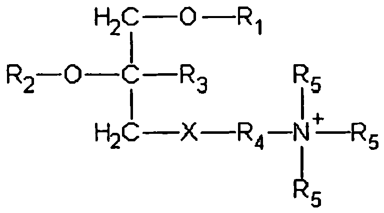 or an enantiomer or diastereomer or a pharmaceutically acceptable salt thereof and at least one pharmaceutically acceptable excipient, wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl;
R₃ is selected from the group consisting of hydrogen and C₁-C₃ alkyl;
R₄ is selected from the group consisting of C₁-C₃ alkyl and C₃-C₆ cycloalkyl; and
R₅ is selected from the group consisting of hydrogen and methyl, and wherein the dosage form is selected from the group consisting of tablets, pills, capsules, granulates, pellets, powders and dragees.

The tri-substituted glycerol compound may be present in amorphous or in crystalline form. The term "amorphous", as used herein, refers to a solid in which there is no long-range order of the positions of the atoms, i.e. a non-crystalline material. In preferred embodiments of the invention, the tri-substituted glycerol compound is present in crystalline form.

The terms "Cₙ alkyl", "Cₙ hydroxyalkyl", and "Cₙ cycloalkyl", as used herein, denote an alkyl group, a hydroxyalkyl group or a cycloalkyl group having n carbon atoms, respectively. For example, the term "C₁₈ alkyl" refers to an alkyl group having 18 carbon atoms. The alkyl groups or hydroxyalkyl groups according to the invention may be straight or branched.

The tri-substituted glycerol compounds of formula (I) have one or more asymmetric centers and thus they can exist as enantiomers or diastereomers. Thus, the pharmaceutical solid dosage forms according to the present invention may comprise either one or more separate individual isomers (such as the L form and the D form) or mixtures of isomers, preferably racemic mixtures.

In some embodiments of the invention, the tri-substituted glycerol compounds of formula (I) are present in the dosage form as pharmaceutically acceptable salts. Such salts may comprise any pharmaceutically acceptable anion "neutralizing" the positive charge of the nitrogen (e.g. chloride, bromide or iodide) or any pharmaceutically acceptable cation "neutralizing" the negative charge of the phosphate or sulfate moiety (e.g. sodium or potassium cations).

In a particular preferred embodiment of the present invention, the pharmaceutical solid dosage form comprises a tri-substituted glycerol compound according to formula (I), wherein X is phosphate, R₁ is -(CH₂)₁₇-CH₃, R₂ is CH₃, R₃ is H, R₄ is - (CH₂)₂-, and R₅ is CH₃.

According to the present invention, it is to be understood that the tri-substituted glycerol compound is present in the pharmaceutical solid dosage form in any amount being effective to achieve the desired pharmacological effect such as to stop tumor progression or to induce an apoptotic effect in tumor cells when administered to a patient. Effective amounts are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention.

Typically, in the pharmaceutical dosage form according to the present invention the amount of the tri-substituted glycerol compound according to formula (I) is less than 400 mg, preferably it is in the range of 30 to 250 mg, and most preferably it is in the range of 50 to 150 mg. In particularly preferred embodiments of the invention, the amount of the tri-substituted glycerol compound according to formula (I) is 75 mg and 100 mg, respectively.

The daily dosage of the tri-substituted glycerol compound administered to a patient is less than 1200 mg, typically less than 900 mg, preferably in the range of 30 to 600 mg, more preferably in the range of 40 to 400 mg, and most preferably in the range of 50 to 350 mg. In specific embodiments, the daily dosage is 75, 100, 150, 200, 225, and 300 mg. Preferably, the daily dosage of the tri-substituted glycerol compound is administered as a single dose such as in form of one up to four tablets or capsules. However, it may also be possible to administer the compound in multiple doses such as two or three individual doses administered during the day, e.g. in the morning, at noon, and at night.

Typically, the pharmaceutical solid dosage form according to the present invention has a total weight of at last 1600 mg. Preferably, the total weight of the dosage form is in the range of 200 to 1200 mg, more preferably in the range of 250 to 1000 mg and most preferably in the range of 300 to 800 mg. The diameter of the solid dosage form is typically at last 17 mm. Preferably, the diameter of the dosage form is in the range of 9 to 15 mm, and particularly preferably in the range of 11 to 12 mm.

The tri-substituted glycerol compound according to formula (I) may be present in the pharmaceutical solid dosage form as a single active ingredient or in combination with at least one other active ingredient such as chemotherapeutics or monoclonal antibodies.

Furthermore, it is known that the absorption and bioavailability of any particular therapeutic agent can be affected by numerous factors when dosed orally. Such factors include the presence of food in the gastrointestinal (GI) tract because, in general, the gastric residence time of a drug is usually significantly longer in the presence of food than in the fasted state. If the bioavailability of a drug is affected beyond a certain point due to the presence of food in the GI tract, the drug is said to exhibit a "food effect" or show a drug/food interaction. This factor should be taken into consideration when choosing the dose amount.

The term "pharmaceutically acceptable excipient" in the meaning of the present invention can be any substance used for the preparation of pharmaceutical dosage forms such as coating materials, film-forming materials, fillers, disintegrating agents, release-modifying materials, carrier materials, diluents, binding agents and other adjuvants.

The term "pharmaceutical solid dosage form for oral application" according to the present invention refers to tablets, pills, capsules, granulates, pellets, powders and dragees.

All these dosage forms are well established in the art (see, e.g., Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL).

In preferred embodiments of the invention, the pharmaceutical solid dosage form is selected from the group consisting of tablets, pills, capsules, and granules, with tablets being particularly preferred.

In another preferred embodiment of the invention, the solid dosage form is an enteric dosage form, i.e. the dosage form remains stable in the stomach, i.e. in an highly acidic environment. This may be achieved by providing a solid dosage form comprising a film coating.

Thus, in further embodiments of the invention, the solid dosage form comprises a film coating. For example, the inventive dosage form may be in the form of a so-called film tablet. The inventive dosage may comprise two or more film coating layers. The corresponding dosage form may be a bilayer or multilayer tablet. The film coating may have a thickness of about 20 microns to about 1200 microns.

Methods for the preparation of film coated dosage forms are well established in the art (see, for example, Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL). It is also well-known in the art how to provide film coatings with specific properties, like enteric coatings, film coating which dissolve upon contact with body fluids, controlled release coatings, taste-masking coatings or disintegrating coatings. In a particularly preferred embodiment, the solid dosage form of the invention comprises an enteric coating.

Typically, the film coating comprises at least one film forming material in an amount of up to 85 % (w/w), based on the total weight of the film coating. In preferred embodiments, the film forming material is selected from the group consisting of acrylic resins such as Eudragit™ polymers (Röhm GmbH &Co. KG, Darmstadt, Germany), polymethacrylate derivatives, gelatin, polyvinyl pyrrolidone, methylcellulose, ethylcellulose, carboxy methylcellulose, hydroxypropyl cellulose, hydroxypropyl methylcellulose, hydroxypropyl methylcellulose acetate succinate, hydroxypropyl methylcellulose phthalate, cellulose acetate phthalate, and polyvinyl acetate phthalate or mixtures thereof, with hydroxypropyl methylcellulose acetate succinate, hydroxypropyl methylcellulose phthalate, cellulose acetate phthalate, polyvinyl acetate phthalate acrylic resins and Eudragit™ polymers being particularly preferred. Preferred Eudragit™ polymers are selected from the group consisting of Eudragit™ L30 D-55, L100-55, L100, L12.5, S100, and S12.5.

The film coating or film coating material according to the present invention may comprise at least one plasticizer. The amount of plasticizer in the film coating material is typically in the range of about 3% (w/w) to 30% (w/w), based on the total weight of the film coating. Suitable plasticizers according to the present invention are selected from the group comprising polyethylene glycol, polyethylene oxide, and triethyl citrate.

The film coating may also comprise at least one stabilizer. Usually, stabilizers are wetting agents such as sorbitol, polyethylene glycol, polyvinyl pyrrolidon or detergents such as sodium lauryl sulfate, e.g. Texapon K12 (Cognis Deutschland GmbH & Co. KG, Düsseldorf, Germany). The stabilizer is typically contained in the film coating material in an amount of about 1% (w/w) to 5% (w/w), based on the total weight of the film coating.

Furthermore, the film coating may also comprise at least one separating agent or anti-adherent. Usually, separating agents are inert compounds such as magnesium/aluminum silicate or metal soaps such as talcum and magnesium stearate. Usually, the amount of separating agent in the film coating material is in the range of about 1% (w/w) to 5% (w/w), based on the total weight of the film coating.

Optionally, the film coating may also comprise pigments for coloring such as titanium oxide, red ferrous oxide or yellow ferrous oxide. Typically, such pigments are present in the film coating material in an amount of up to 1% (w/w), based on the total weight of the coating.

In another preferred embodiment of the present invention, the enteric film coating of the pharmaceutical solid dosage form is soluble at a pH ≥ 6.8, preferably at a pH ≥ 5.5. It is also preferred that the pharmaceutical solid dosage form according to U.S. Pharmacopoeia XXIX <701> disintegrates at a pH in the range of ≥ 6.8 within a contact time of at last 30 minutes (i.e. when in contact with intestinal fluid), preferably within a contact time of at last 15 minutes.

It is further preferred that the pharmaceutical solid dosage form according to U.S. Pharmacopoeia XXIX <701> does not disintegrate at a pH in the range of ≤ 2.5 within a contact time of at least 120 minutes (i.e. when in contact with gastric fluid).

According to the present invention, the solid dosage form may comprise up to 50 % (w/w) of the at least one excipient, wherein the excipient preferably comprises at least one filler, at least one binder, at least one disintegrating agent, at least one flowability-controlling agent, and at least one lubricant

The term "filler", as used herein, refers to inert compounds that may be present in the pharmaceutical solid dosage form of the invention in an amount of up to 70% (w/w), based on the total weight of the dosage form. Examples of suitable filler include *inter alia* lactose, glucose, fructose, calcium hydrogenphosphate (dihydrate), pectin, alginate, starch (e.g., corn starch), microcrystalline cellulose as well as 1:1 mixtures each two of lactose, calcium hydrogenphosphate, microcrystalline cellulose, and corn starch, respectively.

The term "binder", as used herein, refers to an excipient, which is suitable for binding other components to one another. Suitable binders include *inter alia* glucose, dextrin, maltodextrin, methylcellulose, ethylcellulose, hydroxyethyl cellulose, magnesium aluminium silicate, guar gum, polyvinyl pyrrolidone, polyethylene oxide, gelatin, sodium alginate and hydrogenated vegetable oils. Such binders may be present in the dosage form of the invention in an amount of 1% (w/w) to 15% (w/w), based on the total weight of the dosage form.

In addition, the inventive dosage form may also contain one or more lubricants (glidants) such as magnesium stearate, sodium stearylfumarate, stearic acid, and glyceryl palmitostearate in an amount of up to 1% (w/w) based on the total weight of the dosage form.

The dosage form may further comprise at least one disintegrating agent such as cross-linked sodium carboxymethyl cellulose (croscarmellose sodium), cross-linked polyvinyl pyrrolidone, corn starch, and sodium glycol starch. Such disintegrating agents may be present in the dosage form in an amount in the range of 0.5% (w/w) to 4% (w/w), based on the total weight of the dosage form.

The inventive dosage form may also comprise one or more flowability-controlling agents. In preferred embodiments of the invention, the flowability-controlling agent is selected from the group consisting of disperse or colloidal silicon dioxide such as Aerosil™ 200 or Syloid™ 244 (both of Degussa AG, Düsseldorf, Germany), magnesium stearate, calcium arachinate, cetyl alcohol, myristyl alcohol, and mixtures thereof, with silicon dioxide being particularly preferred. Such flowability-controlling agents may be present in the dosage form in an amount of up to 1% (w/w), based on the total weight of the dosage form.

In particularly preferred embodiments of the present invention, the ratio between the tri-substituted glycerol compound and the at least one flowability-controlling agent is 1 part by weight of the tri-substituted glycerol compound to 0.01-0.1 parts by weight of the flowability-controlling agent

Furthermore, it may be desirable to provide controlled release dosage forms that release the tri-substituted glycerol compounds of the invention at a constant rate over a defined period of time. A range of matrix forming natural and synthetic polymers is available to prolong or modify drug release, like for example, xanthan gum, galactomannan polymers, alginate, cellulose derivatives (methycellulose, hydroxypropyl cellulose and hydroxypropyl methylcellulose etc.), acrylic and methacrylic co-polymers and combinations thereof. This range of polymers enables formulators to obtain the desired release profile.

Alternatively, the inventive dosage form may contain one or more excipients which are suitable for regulating or modifying the release of the tri-substituted glycerol compounds of the invention. Suitable excipients for regulating or modifying the release of the tri-substituted glycerol form are hydrophobic release controlling agents and/or hydrophilic polymers.

The hydrophobic release controlling agents may preferably be selected from the group comprising ammonium methacrylate copolymers, methacrylic acid copolymer, polyacrylate, polyvinyl acetate, ethylcellulose, cellulose acetate, cellulose propionate, cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl actylate), poly(octadecyl acrylate), waxes, fatty alcohols, fatty acid esters and hydrogenated castor oil.

The inventive dosage form may also contain an extended release polymer layer with a hydrophilic polymer being selected from the group comprising carboxymethyl cellulose, guar gum, hydroxyethyl cellulose, hydroxypropyl cellulose, hydroxypropylmethyl cellulose, methylcellulose and povidone. Alternatively, the dosage form may contain an extended release polymer layer with a hydrophobic material being selected from the group consisting of carnauba wax, ethylcellulose, glyceryl palmitostearate, hydrogenated castor oil, hydrogenated vegetable oil, microcrystalline wax, polymethacrylate and stearic acid.

In a particularly preferred embodiment of the present invention, the pharmaceutical solid dosage form provides for immediate release of the tri-substituted glycerol compound upon being dissolved and/or disintegrated. It is desirable that the inventive pharmaceutical dosage form provides a defined, preferably rapid release profile. More precisely, the inventive dosage form may be formulated such that at least 80%, preferably at least 85%, of the total amount of the tri-substituted glycerol compound comprised in the dosage form is released from the dosage form within 45 minutes, preferably within 30 minutes, when measured in a type 1 dissolution apparatus (paddle) according to U.S. Pharmacopoeia XXIX <724> at 37°C ± 0.5°C in buffer state at pH 6.8 and 75 rotations per minute and/or may be formulated such that not more than 10% of the total amount of the tri-substituted glycerol compound comprised in the dosage form is released from the dosage form within two hours when measured in a type 1 dissolution apparatus (paddle) according to U.S. Pharmacopoeia XXIX <724> at 37° ± 0.5°C in acidic state at pH 1.2 and 75 rotations per minute.

According to another aspect of the present invention, the active agent, the tri-substituted glycerol compound may be contained or dispersed in a matrix being part of the dosage form. The matrix of the inventive dosage form may preferably be an immediate release matrix, although also normal release or controlled release matrices having a coating that controls the release of the drug may be used.

Suitable materials for a controlled release matrix or coating comprise:
(i) Hydrophilic polymers, such as gums, cellulose ethers, acrylic resins and protein derived materials. Of these polymers, the cellulose ethers, especially hydroxyalkylcelluloses and carboxyalkylcelluloses, are preferred. The dosage form may comprise between 1% and 80% (by weight) of at least one hydrophilic or hydrophobic polymer.
(ii) Digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils and waxes. Hydrocarbons having a melting point of between 25°C and 90°C are preferred. Fatty (aliphatic) alcohols are particularly preferred. The dosage form may comprise up to 60% (by weight) of at least one digestible, long chain hydrocarbon.
(iii) Polyalkylene glycols. The dosage form may comprise up to 60% (by weight) of at least one polyalkylene glycol.

Alternatively, the inventive dosage form may comprise a normal release matrix having a coat that controls the release of the tri-substituted glycerol compound. In some embodiments of the invention, the dosage form may comprise film coated spheroids or granules comprising the tri-substituted glycerol compound and a non-water soluble spheronising agent. The term "spheroid" is known in the pharmaceutical art and denotes a spherical granule having a diameter of between 0.5 mm and 2.5 mm especially between 0.5 mm and 2 mm.

According to another aspect of the present invention, the dosage form may be a multi-particulate containing formulation. The unit doses of multi-particulates may then be incorporated into a pharmaceutical solid dosage formulation, e.g. via compression or shaping into tablets or by placing a requisite amount inside a gelatin capsule. The multi-particulate dosage forms may comprise coated microparticles, like crystals, granules, pellets or beads.

In a second aspect, the present invention relates to a tri-substituted glycerol compound as defined herein for use as a pharmaceutical solid dosage form for oral administration.

In preferred embodiments, the tri-substituted glycerol compound is for the treatment of cancer or for the treatment of immune diseases (cf. also the definitions indicated below).

In a third aspect, the present invention relates to a method for preparing a pharmaceutical solid dosage form as defined herein, the method comprising mixing the tri-substituted glycerol compound with the at least one excipient.

In one embodiment of the invention, the method further comprises drying the mixture. In another embodiment, the method further comprises granulating the mixture obtained.

In a preferred embodiment of the invention relating to the preparation of tablets, the method further comprises compressing the, optionally granulated, mixture by using a suitable tablet press. It is particularly preferred to perform compression of the granulate at a pressure of at last 200 MPa.

Preferably, the at least one excipient comprising fillers, binders, disintegrating agents flowability-controlling agents, lubricants and/or other additives are present in pre-grained form. It is well known to a person of skill in the art how to prepare such pre-grained additives (cf. also the references cited below).

The manufacture of the pharmaceutical solid dosage forms typically occurs at a temperature between 15°C and 26°C, preferably between 18°C and 22°C. The relative humidity in the production rooms is less than 55%, preferably less than 40%.

In another preferred embodiment of the invention, the residual moisture of the final mixture after drying and/or granulating is less than 1.5% (w/w), particularly preferably less than 1.0% (w/w), most preferably less than 0.5% (w/w) based on the total weight of the mixture, respectively.

The method according to the present invention is also preferred to comprise coating the pharmaceutical formulation obtained with a film coating material, particularly preferably with an enteric film coating material.

Methods for preparing pharmaceutical solid dosage forms according to the present invention are well known in the art (see, for example, Gennaro, A.L. and Gennaro, A.R (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Stricker, H. (2003) Arzneiformenentwicklung, Springer Verlag, Berlin, Germany; Niazi, S.K. (2004) Handbook of Pharmaceutical Manufacturing Formulations, CRC Press, Boca Raton, FL).

In a forth aspect, the invention relates to the use of the pharmaceutical solid dosage form, as defined herein, as a medicament for the treatment of cancer or the treatment of immune diseases.

The term "cancer", as used herein, denotes any type or form of malignant growth of cells or tissues including *inter alia* breast cancer, colorectal cancer, prostate cancer, leukemia, lymphomas, melanoma, and lung cancer. Within the scope of the present invention, the term "cancer" refers to a group of diseases in which cells are aggressive (i.e. they grow and divide regardless of normal limits), invasive (i.e. they invade and destroy adjacent tissues), and metastatic (i.e. they spread to other locations in the body). These three "malignant properties" of cancers differentiate them from benign tumors which are self-limited in their growth and do not invade or metastasize (although some benign tumor types are capable of becoming malignant).

The term "immune disease", as used herein, refers to any disorder of the immune system. Examples of such immune diseases include *inter alia* immunodeficiencies (i.e. congenital or acquired conditions in which the immune system's ability to fight infectious diseases is compromised or entirely absent such as AIDS or SCID), hypersensitivity (such as and forms of allergies or asthma), and autoimmune diseases. The term "autoimmune disease" is to be understood to denote any disorder arising from an overactive immune response of the body against endogenic substances and tissues, wherein the body attacks its own cells. Examples of autoimmune diseases include *inter alia* multiple sclerosis, Crohn's disease, lupus erythematosus, myasthenia gravis, rheumatoid arthritis, and polyarthritis.

The invention is further described by the following figures and examples, which are solely for the purpose of illustrating specific embodiments of this invention, and are not to be construed as limiting the scope of the invention in any way.

Materials used in tests below are either commercially available or easily prepared from commercially available materials by those skilled in the art.

### FIGURES

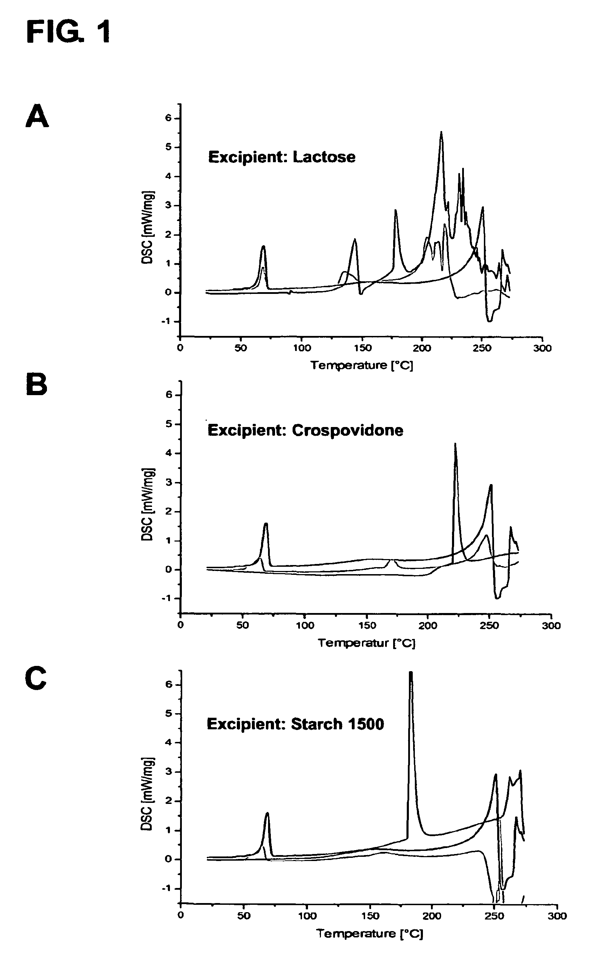

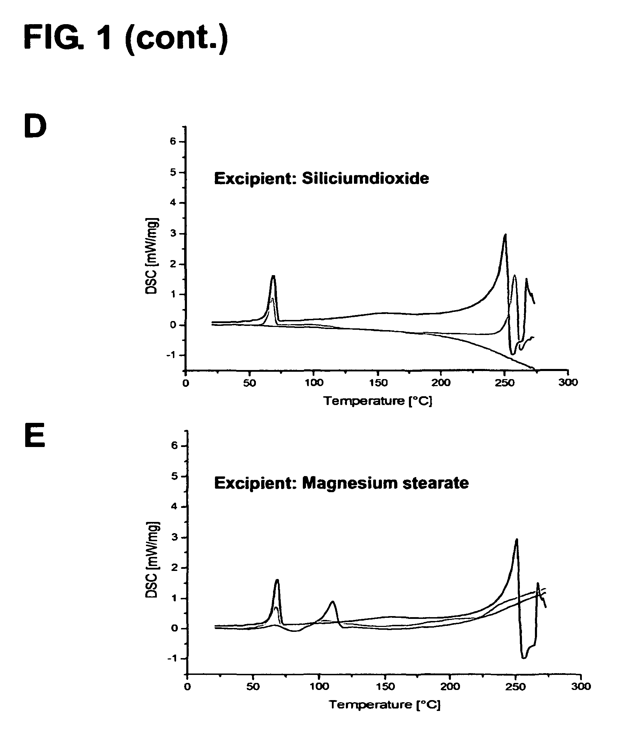

- **Figure 1**: depicts the results of differential scanning calorimetry (DCS) analyses for determining excipient compatibility using a Netzsch DSC 204 apparatus (Netzsch Gerätebau GmbH, Selb, Germany) with a heating rate of 5 K/min up to 300°C and a cooling rate of 1 K/min down to -30°C (temperature onset at room temperature (approx. 20°C)). The below-mentioned samples were tested: crystalline 1-*O*-octadecyl-2-*O*-methyl- glycero-3-phosphocholine alone (black curves), the following excipients alone (red curves): lactose (**Fig. 1A**), crospovidone (**Fig. 1B**), starch 1500 (**Fig. 1C**), siliciumdioxide (**Fig. 1D**), as well as magnesium stearate (**Fig. 1E**), and binary mixtures of 1-*O*-octadecyl-2-*O*-methyl-glycero-3-phosphocholine and each excipient (green curves).

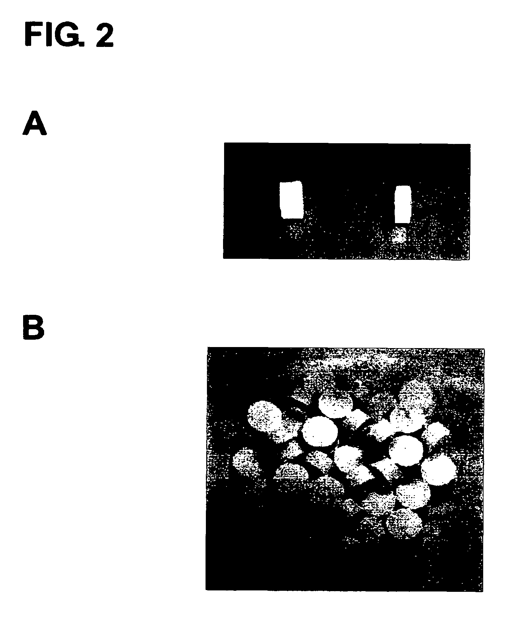

- **Figure 2**: depicts tablets comprising 1-*O*-octadecyl-2-*O*-methyl-glycero-3-phosphocholine according to the present invention. **Fig. 2A** shows granulated tablets prepared as described in example 6. The tablets were compressed in a Korsch eccentric press EKO or XP1 (Korsch AG, Berlin, Germany) in different hardness grades (i.e. breaking strength), namely a hardness of 30 N (left) and a hardness of 90 N (right). The amount of 1-*O*-octadecyl-2-*O*-methyl-glycero-3-phosphocholine in the tablets is 20% (w/w) based on the total weight of the tablets. **Fig. 2B** shows tablets obtained by direct compression as described in example 4. The amount of 1-*O*-octadecyl-2-*O*-methyl-glycero-3-phosphocholine in the tablets is 15% (w/w) based on the total weight of the tablets. The tablets were compressed in a Korsch eccentric press EKO or XP1 (Korsch AG, Berlin, Germany) in a hardness grade of 90 N.

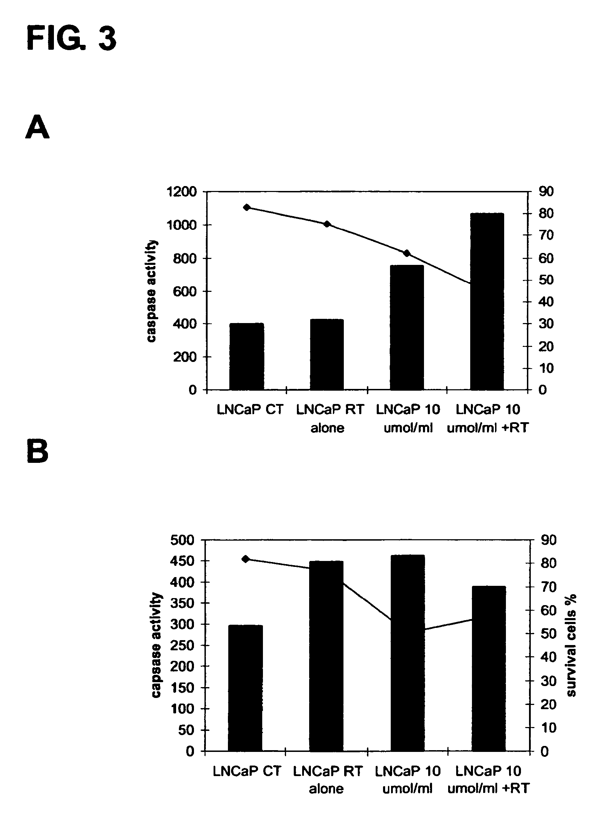

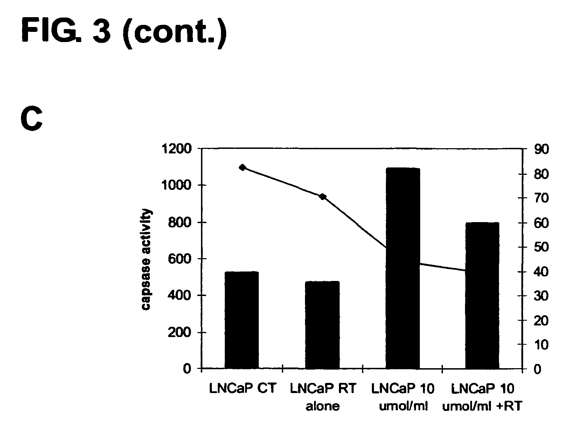

- **Figure 3**: depicts the *in vitro* effects of 1-*O*-octadecyl-2-*O*-methyl-glycero-3-phosphocholine (final concentration 10 µM), (ionizing) radiation (absorbed dose of 5 Gray units; indicated as "RT"), and a combination thereof on programmed cell death (apoptosis) and the survival rate of LNCaP androgen-sensitive human prostate adenocarcinoma cells. Apoptosis was determined using the Apo-ONE™ Homogenous Caspase-3/7 Assay, Promega, Inc., Madison, WI, USA according to the manufacturer's instructions. The percentage of living cancer cells was estimated by means of trypan blue dye exclusion as described (Freshney, R.I. (1994) Culture of Animal Cells: A Manual of Basic Technique. 3rd Ed. Wiley-Liss. New York. USA) The cells were exposed to radiation six hours after (**Fig. 3A**), concomitantly with (**Fig. 3B**), or six hours before administration of 1-*O*-octadecyl-2-*O*-methyl-glycero-3-phospho-choline **(****Fig. 3C**). The caspase assay was performed 12 hours after exposure to radiation. The respective data shown represent the average of two independent experiments.

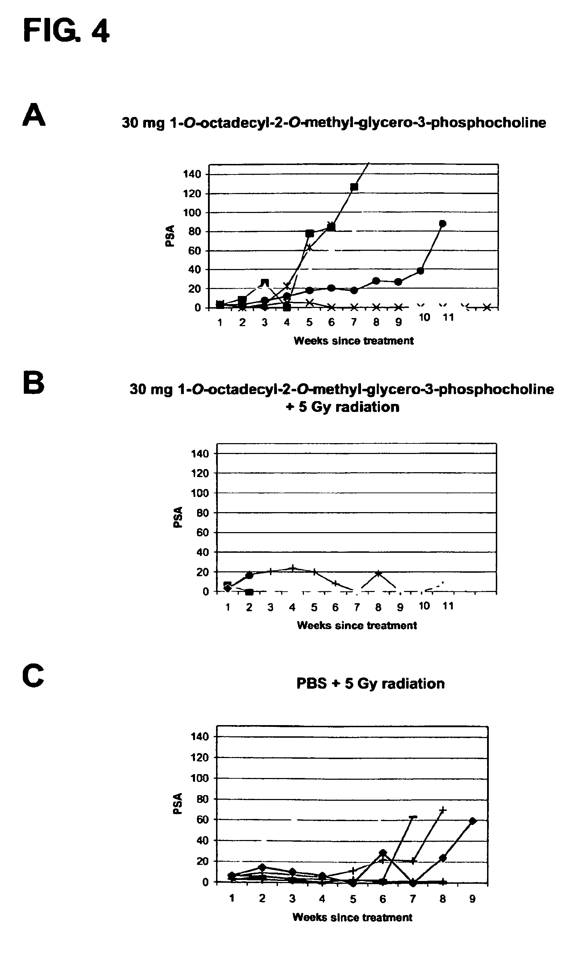

- **Figure 4**: depicts the *in vivo* effects of 1-*O*-octadecyl-2-*O*-methyl-glycero-3-phosphocholine (30 mg/kg body weight/day administered intraperitoneally for 15 days; **Fig. 4A**), (ionizing) radiation (absorbed dose of 5 Gray units administered on day 7; **Fig. 4C**), and a combination thereof (**Fig. 4B**) on LNCaP cells grown orthotopically in the prostates of nude mice (seven mice/group). Tumor growth was assessed via determining the serum level of the prostate-specific antigen (PSA) using a commercially available test kit as well as the tumor volume by means of magnetic resonance imaging.

### EXAMPLES

The methods for preparing the pharmaceutical solid dosage forms according to the present invention follow establish standard methods well known in the pharmaceutical art (see, for example, the following textbooks: Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Stricker, H. (2003) Arzneiformenentwicklung, Springer Verlag, Berlin, Germany; Niazi, S.K. (2004) Handbook of Pharmaceutical Manufacturing Formulations, CRC Press, Boca Raton, FL).

### Example 1: Preparation of an inventive tablet by direct compression

For the preparation of an inventive tablet by direct compression the following ingredients were mixed (per dosage form):

| | |
|---|---|
| 75.0 mg | 1-*O*-octadecyl-2-*O*-methyl-glycero-3-phosphocholine |
| 325.0 mg | lactose |
| 20.0 mg | Kollidon™ VA 64 (BASF, Ludwigshafen, Germany) |
| 4.0 mg | Aerosil™ 200 (Degusta, Düsseldorf, Germany) |
| 2.4 mg | magnesium stearate |
| 6.0 mg | stearic acid |
| 4.0 mg | corn starch |

Subsequently, the mixture was directly compressed in a tablet press.

### Example 2: Preparation of an inventive tablet by direct compression

For the preparation of an inventive tablet by direct compression the following ingredients were mixed (per dosage form):

| | |
|---|---|
| 90.7 mg | 1-*O*-octadecyl-2-*O*-methyl-glycero-3-phosphocholine |
| 1.4 mg | formaldehyde casein |
| 1.4 mg | potato starch |
| 1.4 mg | gel forming agent from red algae |
| 1.4 mg | sodium cellulose glycolate |
| 3.2 mg | stearin talcum |
| 0.5 mg | Aerosil™ 200 (Degusta, Düsseldorf, Germany) |

Subsequently, the mixture was directly compressed in a tablet press.

### Example 3: Preparation of an inventive tablet by direct compression

For the preparation of an inventive tablet by direct compression the following ingredients were mixed (per dosage form):

| | |
|---|---|
| 75.0 mg | 1-*O*-octadecyl-2-*O*-methyl-glycero-3-phosphocholine |
| 200.3 mg | calcium hydrogenphosphate |
| 200.3 mg | microcrystalline cellulose |
| 15.0 mg | Kollidon™ CL (BASF, Ludwigshafen, Germany) |
| 9.4 mg | magnesium stearate |

Subsequently, the mixture was directly compressed in a tablet press.

### Example 4: Preparation of an inventive tablet by direct compression

For the preparation of an inventive tablet by direct compression the following ingredients were mixed (per dosage form):

| | |
|---|---|
| 75.0 mg | 1-*O*-octadecyl-2-*O*-methyl-glycero-3-phosphocholine |
| 196.3 mg | dicalcium phosphate |
| 196.3 mg | Avicel™ PH-102 (FMC BioPolymer, Philadelphia, USA) |
| 20.0 mg | Crospovidone™ (BASF, Ludwigshafen, Germany) |
| 12.4 mg | magnesium stearate |

1-*O*-octadecyl-2-*O*-methyl-glycero-3-phosphocholine, Avicel, and diacalcium phosphate were passed through a sieve having a pore size of IV (about 1 mm) and thoroughly mixed. Crospovidone and magnesium stearate were also sieved and admixed. Subsequently, the tablets were compressed in a Korsch eccentric press EKO or XP1 (Korsch AG, Berlin, Germany; the compression forces used were between 5 kN and 20 kN) in a hardness grade (i.e. breaking strength) of 90 N.

The amount of 1-*O*-octadecyl-2-*O*-methyl-glycero-3-phosphocholine in the tablets is 15% (w/w) (i.e. 75 mg) based on the total weight of the tablets (i.e. 500 mg). Analogously, tablets having a total weight of, for example, 300 mg, 350 mg, 375 mg, 400 mg, and 450 mg were prepared (not shown). The tablets have an average diameter of about 12 mm and an average thickness of about 3 mm to about 5 mm. The tablets obtained are shown in Fig 2B.

### Example 5: Preparation of an inventive granulated tablet

For the preparation of an inventive granulated tablet the following ingredients were mixed and granulated (per dosage form):

| | |
|---|---|
| 75.0 mg | 1-*O*-octadecyl-2-*O*-methyl-glycero-3-phosphocholine |
| 357.7 mg | lactose |
| 40.0 mg | Polyplasdone™ XL (BASF, Ludwigshafen, Germany) |
| 2.4 mg | magnesium stearate |

Subsequently, the mixture was compressed in a tablet press.

### Example 6: Preparation of an inventive granulated tablet

For the preparation of an inventive granulated tablet the following ingredients were mixed and granulated (per dosage form):

| | |
|---|---|
| 75.0 mg | 1-*O*-octadecyl-2-*O*-methyl-glycero-3-phosphocholine |
| 249.5 mg | microcrystalline cellulose |
| 12.5 mg | Kollidon™ 25 (BASF, Ludwigshafen, Germany) |
| 30.0 mg | Crospovidone™ (BASF, Ludwigshafen, Germany) |
| 8.0 mg | magnesium stearate |

1-*O*-octadecyl-2-*O*-methyl-glycero-3-phosphocholine, and microcrystalline cellulose were passed through a sieve having a pore size of IV (about 1 mm) and thoroughly mixed. Kollidon was added as a 20% (w/v) solution in isopropanol and granulated. The granulated mixture (representing the inner phase of the tablet) was dried to a residual moisture of less than 3% (w/w) based on the total weight of the mixture (determined using the Ohaus Moisture Analyzer (Ohaus Corp., Pine Brook, NJ, USA).

Crospovidone and magnesium stearate (representing the outer phase of the tablet) were added. Subsequently, the tablets were compressed in a Korsch eccentric press EKO or XP1 (Korsch AG, Berlin, Germany; the compression forces used were between 5 kN and 20 kN) in different hardness grades (i.e. breaking strength) of 30 N (left) and 90 N (right).

The amount of 1-*O*-octadecyl-2-*O*-methyl-glycero-3-phosphocholine in the tablets is 20% (w/w) based on the total weight of the tablets (i.e. 300-350 mg). Analogously, tablets having a total weight of, for example, 400 mg, 450 mg, 500 mg, 600 mg, 700 mg, and 750 mg were prepared (not shown). The tablets have an average diameter of about 12 mm and an average thickness of about 3 mm to about 5 mm depending on the hardness grade. The tablets obtained are shown in Fig 2A.

### Example 7: Preparation of an inventive granulated tablet

For the preparation of an inventive granulated tablet the following ingredients were mixed and granulated (per dosage form):

| | |
|---|---|
| 150.0 mg | 1-*O*-octadecyl-2-*O*-methyl-glycero-3-phosphocholine |
| (30.0% w/w) | |
| 158.8 mg | FlowLac™ 100 (Meggle Pharma, Wasserburg, Germany) |
| (31.5% w/w) | (lactose monohydrate) |
| 158.8 mg | Avicel™ PH-102 (FMC BioPolymer, Philadelphia, USA) |
| (31.5% w/w) | (microcrystalline cellulose) |
| 20.0 mg | Crospovidone™ (BASF, Ludwigshafen, Germany) |
| (4.0% w/w) | |
| 12.4 mg | magnesium stearate |
| (3.0% w/w) | |

The tablets were prepared in analogy to example 6. The amount of 1-*O*-octadecyl-2-*O*-methyl-glycero-3-phosphocholine in the tablets is 30% (w/w) based on the total weight of the tablets (i.e. 500 mg). The tablets have an average hardness degree (i.e. breaking strength) of about 85 N, an average diameter of about 12 mm and an average thickness of about 5.6 mm.

For the preparation of an alternative inventive granulated tablet the following ingredients were mixed and granulated (per dosage form):

| | |
|---|---|
| 150.0 mg | 1-*O*-octadecyl-2-*O*-methyl-glycero-3-phosphocholine |
| (25.0% w/w) | |
| 205.5 mg | FlowLac™ 100 (Meggle Pharma, Wasserburg, Germany) |
| (34.0% w/w) | (lactose monohydrate) |
| 205.5 mg | Avicel™ PH-102 (FMC BioPolymer, Philadelphia, USA) |
| (34.0% w/w) | (microcrystalline cellulose) |
| 24.0 mg | Crospovidone™ (BASF, Ludwigshafen, Germany) |
| (4.0% w/w) | |
| 15.0 mg | magnesium stearate |
| (3.0% w/w) | |

The tablets were prepared in analogy to example 6. The amount of 1-*O*-octadecyl-2-*O*-methyl-glycero-3-phosphocholine in the tablets is 25% (w/w) based on the total weight of the tablets (i.e. 600 mg). The tablets have an average hardness degree (i.e. breaking strength) of about 86 N, an average diameter of about 13 mm and an average thickness of about 6.1 mm.

### Example 8: Preparation of an inventive granulated tablet

For the preparation of an inventive granulated tablet the following ingredients were mixed and granulated (per dosage form):

| | |
|---|---|
| 20.0% (w/w) | 1-*O*-octadecyl-2-*O*-methyl-glycero-3-phosphocholine |
| ad 100.0% (w/w) | lactose |
| 9.2% (w/w) | Avicel™ PH-101 (FMC BioPolymer, Philadelphia, USA) |
| 15.0% (w/w) | saccharose |
| quantum satis | 15% (w/v) gelatine |
| 0.5% (w/w) | magnesium stearate |
| 2.0% (w/w) | sodium glycol starch |

Subsequently, the mixture was compressed in a tablet press.

### Example 9: Preparation of an inventive granulated tablet

For the preparation of an inventive granulated tablet the following ingredients were mixed and granulated (per dosage form):

| | |
|---|---|
| 20.0% (w/w) | 1-*O*-octadecyl-2-*O*-methyl-glycero-3-phosphocholine |
| ad 100.0% (w/w) | lactose |
| 30.0% (w/w) | Avicel™ PH 101 (FMC BioPolymer, Philadelphia, USA) |
| 10.0% (w/w) | corn starch |
| quantum satis | 10% (w/v) Kollidon™ 25 (BASF, Ludwigshafen, Germany) |
| 1.0% (w/w) | Kollidon™ CL (BASF, Ludwigshafen, Germany) |
| 2.0% (w/w) | magnesium stearate |

Subsequently, the mixture was compressed in a tablet press.

### Example 10: Preparation of an inventive granulated tablet

For the preparation of an inventive granulated tablet the following ingredients were mixed and granulated (per dosage form):

| | |
|---|---|
| 75.0 mg | 1-*O*-octadecyl-2-*O*-methyl-glycero-3-phosphocholine |
| 275.0 mg | lactose |
| 25.0 mg | corn starch |
| quantum satis | 10% (w/v) Kollidon™ 25 (BASF, Ludwigshafen, Germany) |
| 1.5 mg | Aerosil™ 200 (Degusta, Düsseldorf, Germany) |
| 3.0 mg | magnesium stearate |

Subsequently, the mixture was compressed in a tablet press.

### Example 11: Preparation of an inventive granulated tablet

For the preparation of an inventive granulated tablet the following ingredients were mixed and granulated (per dosage form):

| | |
|---|---|
| 75.0 mg | 1-*O*-octadecyl-2-*O*-methyl-glycero-3-phosphocholine |
| 250.0 mg | lactose |
| 25.0 mg | sodium glycol starch |
| quantum satis | 10% (w/v) Kollidon™ 25 (BASF, Ludwigshafen, Germany) |
| 0.5 mg | Aerosil™ 200 (Degusta, Düsseldorf, Germany) |
| 0.5 mg | magnesium stearate |

Subsequently, the mixture was compressed in a tablet press.

### Example 12: Preparation of enteric pellets according to the invention

For the preparation of enteric pellets the following ingredients were mixed and granulated (per dosage form):

| | |
|---|---|
| 75.0 mg | 1-*O*-octadecyl-2-*O*-methyl-glycero-3-phosphocholine |
| 187.0 mg | Avicel™ PH 101 (FMC BioPolymer, Philadelphia, USA) |
| 13.0 mg | GranuLac™ 140 (Meggle Pharma, Wasserburg, Germany) |
| 35.0 mg | Eugradit™ L30 D-55 |
| | (Röhm GmbH &Co. KG, Darmstadt, Germany) |
| 3.4 mg | triethyl citrate |
| 17.0 mg | magnesium stearate |

### Example 13: Preparation of enteric tablets according to the invention

The tablets prepared according to Examples 1 to 9 were coated with an enteric film coating using established standard methods well known in the art (see the references cited above). The following film coatings were used (the amounts of the respective ingredients are given in mg for tablets having a total weight of 300, 400, 500, 600, 700, and 800 mg, respectively):

### Coating 1:

| **Total weight tablet** | **300** | **400** | **500** | **600** | **700** | **800** |
|---|---|---|---|---|---|---|
| Eudragit™ L30 D-55 | 12 | 16 | 20 | 24 | 28 | 32 |
| Sicopharm™ Yellow 10 (Degussa AG, Düsseldorf, Germany) | 1.75 | 2.3 | 2.95 | 3.5 | 3.95 | 4.6 |
| Kollidon™ K90 | 0.4 | 0.55 | 0.65 | 0.8 | 0.95 | 1.1 |

### Coating 2:

| **Total weight tablet** | **300** | **400** | **500** | **600** | **700** | **800** |
|---|---|---|---|---|---|---|
| Eudragit™ L100 | 12 | 16 | 20 | 24 | 28 | 32 |
| Triethyl citrate | 2.4 | 3.2 | 4.0 | 4.8 | 5.6 | 6.4 |

### Coating 3:

| **Total weight tablet** | **300** | **400** | **500** | **600** | **700** | **800** |
|---|---|---|---|---|---|---|
| Eudragit™ L100 | 12 | 16 | 20 | 24 | 28 | 32 |
| Diethyl citrate | 2.4 | 3.2 | 4.0 | 4.8 | 5.6 | 6.4 |
| Talcum | 2.95 | 3.9 | 4.9 | 5.9 | 6.9 | 7.85 |

### Coating 4:

| **Total weight tablet** | **300** | **400** | **500** | **600** | **700** | **800** |
|---|---|---|---|---|---|---|
| Eudragit™ L100 | 12 | 16 | 20 | 24 | 28 | 32 |
| Triethyl citrate | 1.2 | 1.6 | 2.0 | 2.4 | 2.8 | 3.2 |
| Talcum | 16.8 | 22.4 | 28 | 33.6 | 31.9 | 49.8 |
| Magnesium stearate | 2.4 | 3.2 | 4.0 | 4.8 | 5.6 | 6.4 |
| Titanium dioxide | 7.2 | 9.6 | 12 | 14.4 | 16.8 | 19.2 |
| Yellow pigment E 104 (Degusta AG, Düsseldorf, Germany) | 7.2 | 9.6 | 12 | 14.4 | 16.8 | 19.2 |
| PEG 6000 | 2.4 | 3.2 | 4.0 | 4.8 | 5.6 | 6.4 |

### Coating 5:

| **Total weight tablet** | **300** | **400** | **500** | **600** | **700** | **800** |
|---|---|---|---|---|---|---|
| Eudragit™ L30 D-55 | 12 | 16 | 20 | 24 | 28 | 32 |
| Talcum | 2.95 | 3.9 | 3.95 | 5.9 | 6.85 | 7.8 |
| PEG 6000 | 1.2 | 1.6 | 2.0 | 2.4 | 2.8 | 3.2 |
| Anti-foaming emulsion | 0.14 | 0.19 | 0.23 | 0.28 | 0.33 | 0.38 |

### Coating 6:

| **Total weight tablet** | **300** | **400** | **500** | **600** | **700** | **800** |
|---|---|---|---|---|---|---|
| Hydroxypropyl methylcellulose phtalate | 12 | 16 | 20 | 24 | 28 | 32 |
| Triacetine | 1.7 | 2.35 | 2.85 | 3.45 | 4.0 | 4.55 |

### Coating 7:

| **Total weight tablet** | **300** | **400** | **500** | **600** | **700** | **800** |
|---|---|---|---|---|---|---|
| Cellulose acetate phtalate | 12 | 16 | 20 | 24 | 28 | 32 |
| Triacetine | 2.4 | 3.2 | 4.0 | 4.8 | 5.6 | 6.4 |

### Coating 8:

| **Total weight tablet** | **300** | **400** | **500** | **600** | **700** | **800** |
|---|---|---|---|---|---|---|
| Eudragit™ L30 D-55 | 12 | 16 | 20 | 24 | 28 | 32 |
| Triethyl citrate | 1.2 | 1.6 | 2.0 | 2.4 | 2.8 | 3.2 |
| Talcum | 6 | 8 | 9 | 12 | 14 | 16 |
| Titanium dioxide | 1.2 | 1.6 | 2.0 | 2.4 | 2.8 | 3.2 |
| 1N NaOH | 0.17 | 0.23 | 0.29 | 0.35 | 0.40 | 0.46 |
| Yellow pigment E 104 (Degussa AG. Düsseldorf. Germany) | 0.34 | 0.45 | 0.59 | 0.69 | 0.80 | 0.91 |
| PEG 6000 | 0.51 | 0.69 | 0.86 | 1.04 | 1.20 | 1.37 |

### Example 14: Differential Scanning Calorimetry (DCS)

Differential scanning calorimetry (DCS) analyses were performed for determining the compatibility of different excipients for formulating tablets according to the present invention.

DSC is a thermoanalytical technique in which the difference in the amount of heat required to increase the temperature of a sample and reference is measured as a function of temperature. Both the sample and reference are maintained at nearly the same temperature throughout the experiment. Generally, the temperature program for a DSC analysis is designed such that the temperature increases or decreases linearly as a function of time.

The analyses were carried out employing a Netzsch DSC 204 apparatus (Netzsch Gerätebau GmbH, Selb, Germany). The heating rate used was 5 K/min up to a temperature of 300°C and the cooling rate was 1 K/min down to a temperature of-30°C. Temperature onset occurred at room temperature (approx. 20°C).

The below-mentioned samples were tested: crystalline 1-*O*-octadecyl-2-*O*-methylglycero-3-phosphocholine alone (black curves), the following excipients alone (red curves): lactose (Fig. 1A), crospovidone (Fig. 1B), starch 1500 (Fig. 1C), siliciumdioxide (Fig. 1D), as well as magnesium stearate (Fig. 1E), and binary mixtures of 1-O-octadecyl-2-O-methyl-glycero-3-phosphocholine and each excipient (green curves).

As can be seen, siliciumdioxide does not show any interaction with 1-O-octadecyl-2-O-methyl-glycero-3-phosphocholine (i.e. represents an inert inorganic material), whereas lactose, crospovidone, and starch all show such interactions. Interactions were also detected when using microcrystalline cellulose, calciumphosphate, and croscarmellose as excipients (data not shown). On the other hand, magnesium stearate and sodium stearylfumarate dominate the thermogram.

### Example 15: Effects of 1-O-octadecyl-2-O-methyl-glycero-3-phosphocholine in the treatment of prostate cancer

Prostate cancer is a type of cancer developing in the prostate, a gland in the male reproductive system. Prostate cancer is most often discovered by physical examination or by screening blood tests, such as the PSA (prostate specific antigen) test. The PSA test measures the blood level of prostate-specific antigen, a serine protease similar to kallikrein. Its normal function is to liquefy gelatinous semen after ejaculation, allowing spermatazoa to more easily navigate through the uterine cervix. PSA levels above about 4 ng/ml are generally considered indicative for a risk to develop prostate cancer. However, PSA is not a perfect test and should thus be corroborated by additional analyses such as the detection of cell-associated PCA-3 mRNA in the urine.

The two most common treatments for locally-advanced or high risk prostate cancer are radiation therapy (RT) and androgen deprivation (AD), that is hormonal therapy. The effects of 1-*O*-octadecyl-2-*O*-methyl-glycero-3-phosphocholine alone and in combination with RT, AD or RT+AD on the extent of programmed cell death (apoptosis) and the survival of prostate cancer sells were investigated, respectively.

First, the *in vitro* effects of 1-*O*-octadecyl-2-*O*-methyl-glycero-3-phosphocholine, (ionizing) radiation, and a combination thereof on programmed cell death (apoptosis) and the survival rate of LNCaP androgen-sensitive human prostate adenocarcinoma cells were measured. The LNCaP cell line was established from a metastatic lesion of the adenocarcinoma. The cells were treated with a final concentration of 10 µM 1-*O*-octadecyl-2-*O*-methyl-glycero-3-phosphocholine and (ionizing) radiation corresponding to an absorbed dose of 5 Gray units.

Apoptosis was determined using the Apo-ONE™ Homogenous Caspase-3/7 Assay, Promega, Inc., Madison, WI, USA according to the manufacturer's instructions. The percentage of living tumor cells was estimated by means of trypan blue dye exclusion as described (Freshney, RI. (1994) Culture of Animal Cells: A Manual of Basic Technique. 3rd Ed. Wiley-Liss. New York. USA)

The cells were exposed to radiation six hours after (Fig. 3A), concomitantly with (Fig. 3B), or six hours before administration of 1-*O*-octadecyl-2-*O*-methyl-glycero-3-phospho-choline (Fig. 3C). The caspase assay was performed 12 hours after exposure to radiation. The respective data shown represent the average of two independent experiments.

When 1-*O*-octadecyl-2-*O*-methyl-glycero-3-phosphocholine was administered six hours prior to exposing the cells to radiation the combined treatment resulted in a survival of only about 45% of the tumor cells as compared to about 85% in untreated controls. Accordingly, in the treated cells a significant increase (> 2.5 fold) in apoptotic response was observed (as determined by the caspase-3/7 assay). Individual treatment with radiation or 1-*O*-octadecyl-2-*O*-methyl-glycero-3-phosphocholine resulted in intermediate survival rates of about 75% and about 60%, respectively (Fig. 3A).

In case of a concomitant administration of radiation and 1-*O*-octadecyl-2-*O*-methylglycero-3-phosphocholine to the cells the survival rate was determined to be about 55%, which is in the same range as observed for the individual chemical treatment (about 50%). Exposure of the cells only to radiation resulted in a survival rate of about 80%, which is comparable to the untreated controls (about 86%). Surprisingly, the results of the caspase-3/7 assays were similar for the individual and the combined treatment (Fig. 3B).

When 1-*O*-octadecyl-2-*O*-methyl-glycero-3-phosphocholine was administered six hours after exposing the cells to radiation the combined treatment resulted in a survival of about 40% of the cells, which is in the same range as observed for the individual chemical treatment (about 45%). Exposure of the cells only to radiation resulted in a survival rate of about 70%. The extent of apoptosis observed was about 25% increased in the cells only exposed to the chemical as compared to the cells exposed to the combined treatment (Fig. 3C).

Based on the above results it appears as if administration of 1-*O*-octadecyl-2-*O-*methyl-glycero-3-phosphocholine prior to exposing the cells to radiation results in the most significant effect on cell survival rates and apoptotic response.

In a further approach, 10 µM 1-*O*-octadecyl-2-*O*-methyl-glycero-3-phosphocholine (final concentration; "CHEM") and (ionizing) radiation (5 Gray units; "RT") were administered simultaneously to the LNCaP cells but the subsequent incubation period was extended to 24 hours. Apoptosis was measured using the Apo-ONE™ Homogenous Caspase-3/7 Assay as described above and expressed as relative fluorescence units (RFLU). The percentage of apoptotic cells was determined by flow cytometric analysis of annexin V-PE positive-stained and 7-AAD (7-Amino ActinomycinD) negative-stained cells (both purchased from BD Biosciences, San Jose, CA, USA) according to established standard protocols. The results are summarized in the following table. The data represent means ± SEM from three independent experiments.

| **Treatment** | **% Annexin V-PE pos. cells** | **Caspace-3/7 activity (RFLU)** |
|---|---|---|
| Control | 3.6 ± 0.2 | 193 ± 39 |
| CHEM | 18.6 ± 1.0 | 580 ± 207 |
| RT | 5.0 ± 0.6 | 242 ± 36 |
| CHEM+RT | 31.7 ± 1.0* | 1514 ± 102* |

The statistical significance of the results was calculated using the one-way ANOVA, LSD test. * p < 0.0001 compared to each of the individual treatments CHEM and RT, respectively.

The enhancement of apoptosis in the "CHEM+RT" treated cells was also observed in androgen-insensitive LNCaP C4-2 and LNCaP-Res cells (data not shown).

Next, the interaction of 1-*O*-octadecyl-2-*O*-methyl-glycero-3-phosphocholine administration ("CHEM") and androgen deprivation ("AD") was investigated. LNCaP cells were deprived of androgen for two days by charcoal absorption of serum according to established procedures well known in the art. CHEM was added in a final concentration of 5 µM and 10 µM, respectively. In addition, it was tested whether addition of the synthetic androgen R1881 ("R1881") two days prior to CHEM administration resulted in reversal of the effect.

Apoptosis was measured using the Apo-ONE™ Homogenous Caspase-3/7 Assay as described above and expressed as relative fluorescence units (RFLU). The percentage of apoptotic cells was determined via annexin V-PE/7-AAD staining as described above. The caspase-3/7 assay and annexin staining were performed 22 hours after CHEM treatment. The results are summarized in the following table.

| **Treatment** | **% Annexin V-PE pos. cells** | **Caspase-3/7 activity (RFLU)** |
|---|---|---|
| Contr. | 9.6 | 235 |
| Contr. + 5 µM CHEM | 13.0 | 380 |
| Contr. + 10 µM CHEM | 17.5 | 436 |
| AD | 12.6 | 81 |
| AD + 5 µM CHEM | 15.4 | 126 |
| AD + 10 µM CHEM | 27.4 | 115 |
| AD + R1881 | 7.0 | 130 |
| AD + R1881 + 5 µM CHEM | 14.6 | 453 |
| AD + R1881 + 10 µM CHEM | 21.6 | 962 |

Thus, administration of 1-*O*-octadecyl-2-*O*-methyl-glycero-3-phosphocholine to androgen-deprived LNCaP cells resulted in a dose-dependent significant increase in apoptotic response. Furthermore, this effect was not reversed by adding a synthetic androgen to the medium prior to 1-*O*-octadecyl-2-*O*-methyl-glycero-3-phosphocholine treatment.

Additionally, in a preliminary study the *in vivo* effects of 1-*O*-octadecyl-2-*O*-methylglycero-3-phosphocholine, (ionizing) radiation, and a combination thereof on LNCaP cells grown orthotopically in the prostates of nude mice (seven mice/ group) was investigated.

1-*O*-octadecyl-2-*O*-methyl-glycero-3-phosphocholine was administered intraperitoneally in a dose of 30 mg/kg body weight/day administered intraperitoneally for 15 days (Fig. 4A; studies using different routes of administration such as orally or by gavage are currently under way). The (ionizing) radiation corresponds to an absorbed dose of 5 Gray units administered on day 7 (Fig. 4B). The combined treatment is illustrated in Fig. 4C. Tumor growth was assessed via determining the serum level of the prostate-specific antigen (PSA) using a commercially available test kit as well as the tumor volume by means of magnetic resonance imaging.

As can be seen, the combined treatment resulted in a significant decrease of PSA serum levels as compared to either individual treatment ("PBS" represents phosphate-buffered saline) demonstrating that the *in vitro* results can also be transferred to an *in vivo* setting.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modifications and variations of the inventions embodied therein herein disclosed may be resorted to by those skilled in the art, and that such modifications and variations are considered to be within the scope of this invention.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Pharmaceutical solid dosage form for oral administration comprising a tri-substituted glycerol compound according to formula (I) 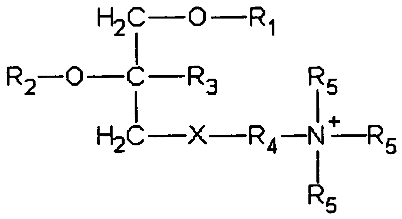 or an enantiomer or diastereomer or a pharmaceutically acceptable salt thereof and at least one pharmaceutically acceptable excipient, wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl;
R₃ is selected from the group consisting of hydrogen and C₁-C₃ alkyl;
R₄ is selected from the group consisting of C₁-C₃ alkyl and C₃-C₆ cycloalkyl; and
R₅ is selected from the group consisting of hydrogen and methyl,
and wherein the dosage form is selected from the group consisting of tablets, pills, capsules, granulates, pellets, powders and dragees.

2. The pharmaceutical solid dosage form according to claim 1, wherein X is phosphate, R₁ is -(CH₂)₁₇-CH₃, R₂ is CH₃, R₃ is H, R₄ is -(CH₂)₂-, and R₅ is CH₃.

3. The pharmaceutical solid dosage form according to claim 1 or 2, wherein the tri-substituted glycerol compound is present in crystalline form.

4. The pharmaceutical solid dosage form according to any of claims 1 to 3, wherein the amount of the tri-substituted glycerol compound is in the range of 30 to 250 mg, preferably in the range of 50 to 150 mg.

5. The pharmaceutical solid dosage form according to any of claims 1 to 4, wherein the dosage form is an enteric dosage form.

6. The pharmaceutical solid dosage form according to any of claims 1 to 5, wherein the dosage form is soluble at a pH ≥ 5.5, preferably at a pH ≥ 6.8.

7. The pharmaceutical solid dosage form according to claim 6, wherein the dosage form according to U.S. Pharmacopoeia XXIX <701> disintegrates at a pH in the range of ≥ 6.8 within a contact time of at last 30 minutes, and preferably does not disintegrate at a pH in the range of ≤ 2.5 within a contact time of at least 120 minutes.

8. The pharmaceutical solid dosage form according to any of claims 1 to 7, wherein the dosage form comprises a film coating, preferably an enteric film coating.

9. The pharmaceutical solid dosage form according to any of claims 1 to 8, wherein the at least one excipient comprises at least one flowability-controlling agent, and wherein preferably the ratio between the tri-substituted glycerol compound and the at least one flowability-controlling agent is 1 part by weight of the tri-substituted glycerol compound to 0.01-0.1 parts by weight of the flowability-controlling agent.

10. The pharmaceutical solid dosage form according to any of claims 1 to 9, wherein the dosage form comprises at least one release-controlling agent.

11. The pharmaceutical solid dosage form according to any of claims 1 to 10, wherein at least 75% of the total amount of tri-substituted glycerol compound comprised in the dosage form is released from the dosage form within 45 minutes when measured in a type 1 dissolution apparatus (paddle) according to U.S. Pharmacopoeia XXIX <724> at 37°C ± 0.5°C in buffer state at pH 6.8 and 75 rotations per minute, and wherein preferably not more than 10% of the total amount of tri-substituted glycerol compound comprised in the dosage form is released from the dosage form within 2 hours when measured in a type 1 dissolution apparatus (paddle) according to U.S. Pharmacopoeia XXIX <724> at 37°C ± 0.5°C in acidic state at pH 1.2 and 75 rotations per minute.

12. Method for preparing a pharmaceutical solid dosage form according to any of claims 1 to 11, comprising:
(a) mixing the tri-substituted glycerol compound with the at least one excipient.

13. The method according to claim 12, further comprising:
(b) drying the mixture obtained in (a); and/or
(c) granulating the mixture obtained in (a) or (b).

14. Method according to claim 12 or 13, wherein the residual moisture of the mixture after performing step (a) and/or (b) and/or (c) is less than 1.5% (w/w) based on the total weight of the mixture.

15. The method according to any of claims 11 to 14, further comprising:
(d) compressing the mixture using a suitable tablet press, wherein compression is preferably performed at a pressure of at last 200 MPa; and/or
(e) coating the pharmaceutical formulation obtained with a film coating material.

16. Use of a pharmaceutical solid dosage form according to any of claims 1 to 11 for the manufacture of a medicament for the treatment of cancer or of immune diseases.

## Patentansprüche

1. Feste pharmazeutische Dosierungsform zur oralen Verabreichung, umfassend eine trisubstituierte Glycerol-Verbindung gemäß der Formel (I) 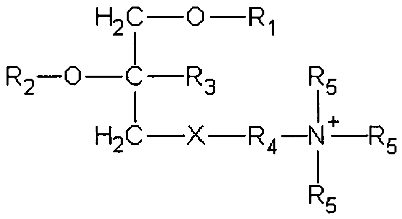 oder eines Enantionmers oder Diastereomers oder eines pharmazeutisch akzeptablen Salzes davon und mindestens einen pharmazeutisch akzeptablen Hilfsstoff, wobei
X ausgewählt ist aus der Gruppe bestehend aus Phosphat und Sulfat;
R₁ ausgewählt ist aus der Gruppe bestehend aus C₁₆-C₂₀-Alkyl;
R₂ ausgewählt ist aus der Gruppe bestehend ausC₁-C₃-Alkyl und C₁-C₃-Hydroxyalkyl;
R₃ ausgewählt ist aus der Gruppe bestehend aus Wasserstoff und C₁-C₃-Alkyl;
R₄ ausgewählt ist aus der Gruppe bestehend aus C₁-C₃-Alkyl und C₃-C₆-Cykloalkyl; und
R₅ ausgewählt ist aus der Gruppe bestehend aus Wasserstoff und Methyl;
und wobei die Dosierungsform ausgewählt ist aus der Gruppe bestehend aus Tabletten, Pillen, Kapseln, Granulen, Pellets, Pulvern und Dragees.

2. Feste pharmazeutische Dosierungsform gemäß Anspruch 1, wobei X Phosphat ist, R₁ -(CH₂)₁₇-CH₃ ist, R₂ CH₃ ist, R₃ H ist, R₄ -(CH₂)₂- ist und R₅ CH₃ ist.

3. Feste pharmazeutische Dosierungsform gemäß Anspruch 1 oder 2, wobei die trisubstituierte Glycerol-Verbindung in kristalliner Form vorliegt.

4. Feste pharmazeutische Dosierungsform gemäß einem der Ansprüche 1 bis 3, wobei die Menge der trisubstituierten Glycerol-Verbindung im Bereich von 30 bis 250 mg, vorzugsweise im Bereich von 50 bis 150 mg ist.

5. Feste pharmazeutische Dosierungsform gemäß einem der Ansprüche 1 bis 4, wobei die Dosierungsform eine enterische Dosierungsform ist.

6. Feste pharmazeutische Dosierungsform gemäß einem der Ansprüche 1 bis 5, wobei die Dosierungsform bei einem pH ≥ 5,5, vorzugsweise bei einem pH ≥ 6,8 löslich ist.

7. Feste pharmazeutische Dosierungsform gemäß Anspruch 6, wobei die Dosierungsform gemäß US-Pharmacopoeia XXIX <701> bei einem pH im Bereich von ≥ 6,8 innerhalb einer Kontaktzeit von mindestens 30 Minuten zerfällt, und vorzugsweise nicht bei einem pH im Bereich von ≤ 2,5 innerhalb einer Kontaktzeit von mindestens 120 Minuten zerfällt.

8. Feste pharmazeutische Dosierungsform gemäß einem der Ansprüche 1 bis 7, wobei die Dosierungsform eine Filmbeschichtung, vorzugsweise eine enterische Filmbeschichtung umfasst.

9. Feste pharmazeutische Dosierungsform gemäß einem der Ansprüche 1 bis 8, wobei der mindestens eine Hilfsstoff mindestens ein die Fließfähigkeit steuerndes Mittel umfasst, und wobei vorzugsweise das Verhältnis zwischen der trisubstituierten Glyercol-Verbindung und dem mindestens einen die Fließfähigkeit steuernden Mittel 1 Gewichtsteil der trisubstituierten Glycerol-Verbindung zu 0,01-0,1 Gewichtsteilen des die Fließfähigkeit steuernden Mittels beträgt.

10. Feste pharmazeutische Dosierungsform gemäß einem der Ansprüche 1 bis 9, wobei die Dosierungsform mindestens ein die freisetzungsteuerndes Mittel umfasst.

11. Feste pharmazeutische Dosierungsform gemäß einem der Ansprüche 1 bis 10, wobei mindestens 75% der Gesamtmenge an trisubstituierter Glycerol-Verbindung umfasst von der Dosierungsform von der Dosierungsform innerhalb von 45 Minuten bei Messung in einer Typ 1-Freisetzungsapparatur(paddle) gemäß US-Pharmacopoeia XXIX <724> bei 37°C ± 0,5°C im gepufferten Zustand bei pH 6,8 und 75 Umdrehungen pro Minute freigesetzt wird, und wobei vorzugsweise nicht mehr als 10% der Gesamtmenge an trisubstituierter Glycerol-Verbindung umfasst von der Dosierungsform von der Dosierungsform innerhalb von 2 Stunden bei Messung in einer Typ 1-Freisetzungsapparatur (paddle) gemäß US-Pharmacopoeia XXIX <724> bei 37°C ± 0,5°C in saurem Zustand bei pH 1,2 und 75 Umdrehungen pro Minute freigesetzt wird.

12. Verfahren zur Herstellung einer festen pharmazeutischen Dosierungsform gemäß einem der Ansprüche 1 bis 11, umfassend:
(a) Mischen der trisubstituierten Glycerol-Verbindung mit dem mindestens einen Hilfsstoff.

13. Verfahren gemäß Anspruch 12, ferner umfassend:
(b) Trocknen des in (a) erhaltenen Gemisches; und/oder
(c) Granulieren der in (a) oder (b) erhaltenden Gemisches.

14. Verfahren gemäß Anspruch 12 oder 13, wobei die Restfeuchte des Gemisches nach Durchführung von Schritt (a) und/oder (b) und/oder (c) weniger als 1,5 Gew.-% basierend auf dem Gesamtgewicht des Gemisches beträgt.

15. Verfahren gemäß einem der Ansprüche 11 bis 14, ferner umfassend:
(d) Komprimieren des Gemisches unter Verwendung einer geeigneten Tablettenpresse, wobei die Kompression vorzugsweise bei einem Druck von mindestens 200 MPa durchgeführt wird; und/oder
(e) Beschichten der erhaltenen pharmazeutischen Formulierung mit einem Filmbeschichtungsmaterial.

16. Verwendung einer festen pharmazeutischen Dosierungsform gemäß einem der Ansprüche 1 bis 11 zur Herstellung eines Medikaments fiir die Behandlung von Krebs oder Immunkrankheiten.

## Revendications

1. Forme galénique pharmaceutique solide destinée à être administrée par voie orale, comprenant un composé de glycérol trisubstitué selon la formule (I) 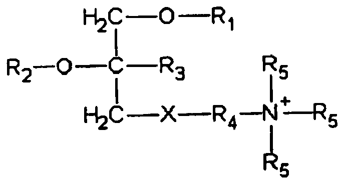 ou un énantiomère ou diastéréomère ou un sel pharmaceutiquement acceptable de ceux-ci et au moins un excipient pharmaceutiquement acceptable, où
X est sélectionné dans le groupe constitué du phosphate et du sulfate ;
R₁ est sélectionné dans le groupe constitué de l'alkyle de C₁₆-C₂₀ ;
R₂ est sélectionné dans le groupe constitué de l'alkyle de C₁-C₃ et de l'hydroxyalkyle de C₁-C₃ ;
R₃ est sélectionné dans le groupe constitué de l'hydrogène et de l'alkyle de C₁-C₃;
R₄ est sélectionné dans le groupe constitué de l'alkyle de C₁-C₃ et du cycloalkyle de C₃-C₆ ; et
R₅ est sélectionné dans le groupe constitué de l'hydrogène et du méthyle,
et où la forme galénique est sélectionnée dans le groupe constitué de comprimés, pilules, capsules, granulés, pastilles, poudres et dragées.

2. La forme galénique pharmaceutique solide selon la revendication 1, où X est du phosphate, R₁ est -(CH₂)₁₇-CH₃, R₂ est CH₃, R₃ est H, R₄ est -(CH₂)₂-, et R₅ est CH₃.

3. La forme galénique pharmaceutique solide selon la revendication 1 ou 2, où le composé de glycérol trisubstitué est présent sous forme cristalline.

4. La forme galénique pharmaceutique solide selon l'une quelconque des revendications 1 à 3, où la quantité de composé de glycérol trisubstitué est comprise dans la plage de 30 à 250 mg, de préférence dans la plage de 50 à 150 mg.

5. La forme galénique pharmaceutique solide selon l'une quelconque des revendications 1 à 4, où la forme galénique est une forme galénique entérique.

6. La forme galénique pharmaceutique solide selon l'une quelconque des revendications 1 à 5, où la forme galénique est soluble à un pH ≥ 5,5, de préférence à un pH ≥ 6,8.

7. La forme galénique pharmaceutique solide selon la revendication 6, où la forme galénique selon la Pharmacopée des Etats-Unis XXIX <701> se désintègre à un pH compris dans la plage de ≥ 6,8 en l'espace d'un temps de contact d'au moins 30 minutes, et de préférence ne se désintègre pas à un pH compris dans la plage de ≤ 2,5 en l'espace d'un temps de contact d'au moins 120 minutes.

8. La forme galénique pharmaceutique solide selon l'une quelconque des revendications 1 à 7, où la forme galénique comprend un pelliculage, de préférence un pelliculage entérique.

9. La forme galénique pharmaceutique solide selon l'une quelconque des revendications 1 à 8, où l'au moins un excipient comprend au moins un agent de contrôle de la coulabilité, et où de préférence le ratio entre le composé de glycérol trisubstitué et l'au moins un agent de contrôle de la coulabilité est de 1 partie en poids du composé de glycérol trisubstitué à 0,01-0,1 partie en poids de l'agent de contrôle de la coulabilité.

10. La forme galénique pharmaceutique solide selon l'une quelconque des revendications 1 à 9, où la forme galénique comprend au moins un agent de contrôle de la libération.

11. La forme galénique pharmaceutique solide selon l'une quelconque des revendications 1 à 10, où au moins 75 % de la quantité totale de composé de glycérol trisubstitué comprise dans la forme galénique est libérée à partir de la forme galénique dans les 45 minutes lorsqu'elle est mesurée dans un appareil de dissolution de type 1 (palette) selon la Pharmacopée des Etats-Unis XXIX <724> à 37°C +/- 0,5°C à un état tampon à un pH de 6,8 et 75 tours par minute, et où de préférence pas plus de 10 % de la quantité totale de composé de glycérol trisubstitué comprise dans la forme galénique est libérée à partir de la forme galénique dans les 2 heures lorsqu'elle est mesurée dans un appareil de dissolution de type 1 (palette) selon la Pharmacopée des Etats-Unis XXIX <724> à 37°C +/-0,5°C à l'état acide à un pH de 1,2 et 75 tours par minute.

12. Procédé d'élaboration d'une forme galénique solide pharmaceutique selon l'une quelconque des revendications 1 à 11, comprenant :
(a) le mélange du composé de glycérol trisubstitué avec l'au moins un excipient.

13. Le procédé selon la revendication 12, comprenant en outre :
(b) le séchage du mélange obtenu en (a) ; et/ou
(c) la granulation du mélange obtenu en (a) ou (b).

14. Procédé selon la revendication 12 ou 13, où l'humidité résiduelle du mélange après réalisation de l'étape (a) et/ou (b) et/ou (c) est inférieure à 1,5 % (poids/poids) sur la base du poids total du mélange.

15. Le procédé selon l'une quelconque des revendications 11 à 14, comprenant en outre :
(d) la compression du mélange à l'aide d'une presse à comprimés adéquate, où la compression est de préférence réalisée à une pression d'au moins 200 MPa ; et/ou
(e) le revêtement de la formulation pharmaceutique obtenue avec une matière de pelliculage.

16. Utilisation d'une forme galénique solide pharmaceutique selon l'une quelconque des revendications 1 à 11 pour la fabrication d'un médicament pour le traitement du cancer ou de maladies immunitaires.
